(19) Europäisches Patentamt
European Patent Office
Office européen des brevets

(11) Numéro de publication : **0 446 116 A1**

(12) **DEMANDE DE BREVET EUROPEEN**

(21) Numéro de dépôt : **91400582.2**

(22) Date de dépôt : **04.03.91**

(51) Int. Cl.$^5$ : **C07D 333/72,** C07C 1/32, C07C 13/19

(30) Priorité : **05.03.90 FR 9002724**

(43) Date de publication de la demande : **11.09.91 Bulletin 91/37**

(84) Etats contractants désignés : **AT BE CH DE DK ES FR GB GR IT LI LU NL SE**

(71) Demandeur : **RHONE-POULENC NUTRITION ANIMALE
Rue Marcel Lingot
F-03600 Commentry (FR)**

(72) Inventeur : **Duchesne Jean-Pierre
160 rue Marcel Mérieux
F-69007 Lyon (FR)**

(74) Mandataire : **Le Pennec, Magali et al
RHONE-POULENC RORER SA, Direction des Brevets, 20 Avenue Raymond Aron
F-92165 Antony Cédex (FR)**

(54) **Nouveaux sulfolènes, leur procédé de préparation et leur utilisation.**

(57) La présente invention concerne de nouveaux dérivés du sulfolène et plus particulièrement le 4,4-diméthyl 2,2-dioxo 1, 2, 4, 5, 6, 7-hexahydrobenzo[c] thiophène.

Ces dérivés sont préparés par mise en contact de la myrcène sulfone avec un acide fort.

Les dérivés de la présente invention sont utilisés comme intermédiaires de synthèse dans la préparation de la vitamine A.

EP 0 446 116 A1

# NOUVEAUX SULFOLENES, LEUR PROCEDE DE PREPARATION ET LEUR UTILISATION

La présente invention concerne de nouveaux dérivés du sulfolène, leur procédé de préparation et leur utilisation dans la préparation des gamma et delta pyronènes et l'utilisation de ces derniers pour la synthèse de la vitamine A. Elle concerne plus particulièrement trois isomères des 4,4-diméthylhexahydrobenzothiophène 2,2-dioxyde.

Ces dérivés répondent à la formule générale suivante :

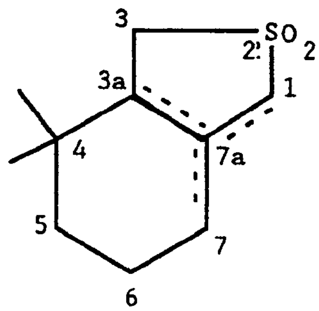

Formule (I)

dans laquelle la ligne discontinue représente une seule double liaison qui peut ainsi être située en position 3a-7a, 1-7a, 7a-7.

Les trois isomères de la présente demande sont ainsi :

– le 4,4-diméthyl 2,2-dioxo 1,3,4,5,6,7-hexahydrobenzo[c] thiophène
– le 4,4-diméthyl 2,2-dioxo 3,3a,4,5,6,7-hexahydrobenzo[c] thiophène
– le 4,4-diméthyl 2,2-dioxo 1,3,3a,4,5,6-hexahydrobenzo[c] thiophène.

On préfère parmi ces isomères le 4,4-diméthyl 2,2-dioxo 1,3,4,5,6,7-hexahydrobenzo[c] thiophène ou isomère β.

Les principaux documents connus dans l'art antérieur décrivant des dérivés de la familles des dioxythiophènes substitués en position 2 du cycle thiophène par une chaîne terpénique sont les brevets US 3.176.022 et US 3.075.003 qui décrivent la sulfonation du myrcène par le dioxyde de soufre suivie de son hydratation en milieu sulfurique et de sa décomposition à la chaleur qui permettent d'obtenir le méthyl-2 méthylène-6 octène-7 ol-2. Ces dérivés sont utilisés en parfumerie.

Les principales méthodes d'accès aux pyronènes sont la deshydratation du cyclogeraniol et la décomposition des sels d'oniums β-cyclogeranyliques comme décrit dans les brevets US 4179468 et US 4244890 pour préparer les gamma et delta pyronènes.

Aucun texte de l'art antérieur ne décrit ni ne suggère des dérivés de l'hexahydrobenzothiophène dioxo-2,2 qui sont d'après la présente invention des intermédiaires de synthèse utiles dans la production de la vitamine A.

Les composés selon l'invention de formule générale (I) sont préparés à partir de la myrcène sulfone ou thiophène 2,5-dihydro 3-(4-méthyl 3-pentényl)1,1- dioxyde de formule (II)

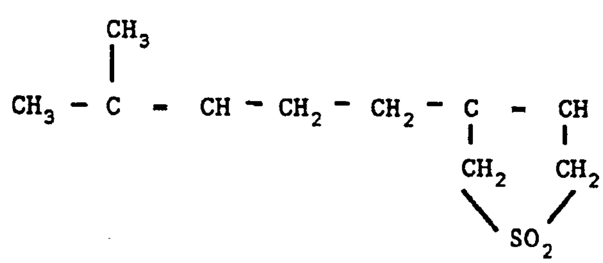

Formule (II)

par cyclisation en présence d'un acide fort contenant moins de 5 % d'eau.

Les acides forts sont choisi notamment parmi :

– l'acide sulfurique
– les acides alkyl, aryl ou halogèno sulfoniques de formule $RSO_3H$ tels que notamment :
  . l'acide méthanesulfonique ($R = CH_3$-)
  . l'acide paratoluènesulfonique ( $R = CH_3C_6H_4$-)
  . l'acide triflique ($R = CF_3$-)

. l'acide fluorosulfonique ( R = F-)
. l'acide chlorosulfonique (R = Cl-)
. les résines sulfoniques telles que l'amberlite 15
– les résines Nafions
– l'acide perchlorique
– les catalyseurs hétérogènes acides tels que :
. les silices acidifiées par un traitement acide (HF...)
. les alumines acidifiées par un traitement acide (HF...)
. les oxydes acides de métaux de transition
. les zéolithes
. les argiles acidifiées.

On préfère utiliser l'acide sulfurique.

L'acide peut être utilisé seul, il est alors réactif et solvant ou en présence d'un solvant inerte dans les conditions de la réaction, miscible avec l'acide et avec les produits de réaction.

Parmi les solvants inertes on peut citer : les solvants halogénés tels que le dichlorométhane, les acides carboxyliques tels que l'acide acétique, leurs esters tels que l'acétate d'éthyle, les solvants nitrés comme le nitrométhane ou le nitrobenzène et les sulfones comme le sulfolane.

En général, lorsqu'un solvant est utilisé, la vitesse de réaction est réduite, il y a aussi apparition d'une quantité plus importante d'isomères α et γ.

Lorsque la réaction est réalisée dans l'acide sulfurique, on préfère utiliser un rapport volumique acide fort à la sulfone de formule (II) inférieur à 1 et de préférence compris entre 0,10 et 0,50.

Lorsqu'on utilise un acide sulfonique, on préfère utiliser un rapport molaire acide sulfonique à la sulfone de formule (II) compris entre 0,1 et 0,5.

Le rapport sera adapté par l'homme de l'art :
– à la vitesse optimale de réaction désirée
– à la nature des isomères recherchés
– à la viscosité du milieu réactionnel.

Les conditions réactionnelles optimales sont telles que la température est de préférence inférieure à 0°C et encore plus préférentiellement comprise entre -10°C et 0°C. La durée de la réaction sera adaptée aux conditions réactionnelles. La mise en oeuvre de l'invention dépend de l'utilisation ou non d'un solvant.

Dans le cas de l'acide sulfurique, on préfère couler la sulfone dans l'acide et arrêter la réaction à la fin de la coulée.

En utilisant, dans les conditions de la réaction, un acide dilué dans un solvant inerte le contact de la sulfone de formule (II) et de l'acide fort peut être prolongé avantageusement au-delà de la coulée de l'acide fort dans la solution de la sulfone.

La matière première mise en oeuvre qui est la myrcène sulfone de formule (II) est préparée par mise en contact du myrcène avec le dioxyde de soufre en présence d'un inhibiteur de polymérisation à une température comprise entre 60 et 100°C tel que décrit notamment dans le brevet US 3.176.022 colonne 1, lignes 50 à 60.

Le myrcène utilisé au départ peut être un produit pur ou un produit brut contenant par exemple des sous-produits terpéniques de synthèse tels que le limonène.

Les produits de formule (I), objet de la présente invention sont des intermédiaires de synthèse de la vitamine A.

Ils sont aisément transformés en δ pyronène par chauffage à haute température éventuellement en présence d'un catalyseur basique selon par exemple la réaction suivante à partir de la β sulfone de formule (I):

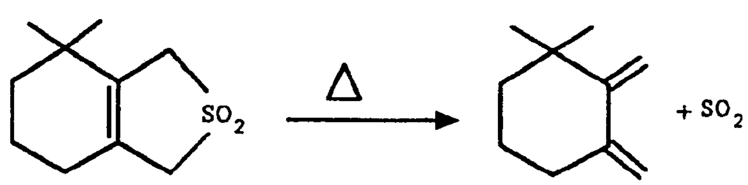


Le catalyseur basique est choisi notamment parmi les hydroxydes alcalins (potasse ou soude), les carbonates, les oxydes métalliques tel que l'alumine, la chaux, lamagnésie ou les alcoolates alcalins tels que le méthylate de sodium ou le décanolate de potassium. On utilise de 10 à 100 % en poids de catalyseur par rapport à la sulfone de formule (I). Lorsque l'on utilise un catalyseur à base d'un oxyde de métal alcalin ou alcalino-terreux, on préfère utiliser 100 % de catalyseur par rapport à la sulfone.

Lorsque l'on utilise un catalyseur à base d'alcoolate alcalin, on préfère utiliser moins de 10 % molaire de

catalyseur par rapport au composé de formule (I).

La température de réaction est de préférence supérieure à 150°C et encore plus préférentiellement comprise entre 250°C et 300°C quand on n'utilise pas de catalyseur et entre 150 et 250°C quand on utilise un catalyseur.

Préalablement à la réaction de transformation de la β sulfone en δ pyronène, losque la matière de départ contient des sulfones α et γ il est facile d'isomériser ces derniers composés en sulfone β par chauffage de quelques minutes à quelques heures à une température comprise entre la température ambiante et 100°C.

La réaction peut être mise en oeuvre en continu ou en discontinu.

Le δ pyronène obtenu après crackage du composé de formule (I) est facilement purifié par distillation.

Le δ pyronène est utilisé dans la synthèse d'intermédiaires cyclogeranyliques utilisables dans la synthèse de la vitamine A et des caroténoïdes tels que décrit par exemple dans le brevet DE 1.025.871 ou dans l'article de K. TAKABE and Coll., Chem. and Ind. (1980) page 540.

Il peut aussi être utilisé comme intermédiaire de synthèse en parfumerie pour préparer l'α-cyclogéraniol.

Les exemples suivants donnés à titre nullement limitatifs permettent une meilleure compréhension de l'invention sans toutefois en limiter la portée.

EXEMPLE 1

Dans un ballon de 100 ml, on refroidit à -10°C sous forte agitation 25 ml $H_2SO_4$ à 98 %. Puis, en 10 mn environ, on coule 6,0 g de sulfolène du myrcène à 95 % obtenu selon l'exemple 1 du brevet US 3.075.003 (28,5 mmole) en maintenant la température au-dessous de 0°C.

La solution brune, visqueuse est agitée 10 mn à 0°C puis coulée lentement sur 100 g d'un mélange eau-glace sous forte agitation.

Le solide blanc qui précipite est filtré, lavé à l'eau jusqu'à neutralité puis séché à 20°C sous 1 mmHg pour donner 5,9 g de produit brut.

Après recristallisation dans 20 ml d'éther isopropylique, on obtient 5,10 g (25,5 mmoles, Rdt = 90 %) de sulfolène du δ pyronène (Ia) sous forme de paillettes blanches PF = 90°C.

Analyse par spectrométrie RMN

Sulfolène (Ia)

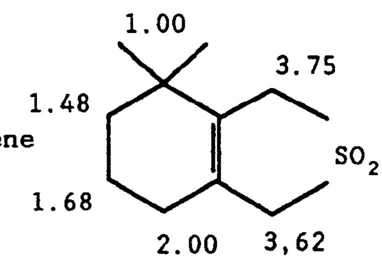

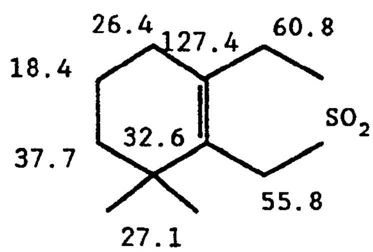

$^{1}H$ RMN 200 MHZ

$^{13}C$

EXEMPLE 2

Dans un ballon de 2 litres muni d'une agitation mécanique puissante, d'une ampoule de coulée et d'un thermomètre, on dissout 500 g de sulfolène du myrcène à 95 % (2,375 moles) dans 100 ml de chlorure de méthylène. En maintenant la température à 0°C on coule en 2 heures une solution de 121 g d'acide méthanesulfonique 98 % dans 100 ml de chlorure de méthylène.

Après 15 mn, on verse sur un mélange de 500 ml eau et de 100 g de glace on extrait avec 500 ml de chlorure de méthylène et 300 ml d'acétate d'éthyle.

Après évaporation des solvants, on obtient 450 g d'une huile brune très épaisse.

Par cristallisation dans 2 l d'éther isopropylique, on obtient 300 g d'un solide blanc qui par chromatographie sur silice permet d'obtenir :

- 240 g de sulfolène Ia PF = 90°C
- 20 g de sulfone Ib PF = 87°C
- 20 g d'un mélange de sulfones $I_b$ et $I_c$.

La structure des sulfones $I_b$ et $I_c$ a été déterminée par RMN dans $CHCl_3$ ref. HMD

$^{1}$HRMN 360 MHZ

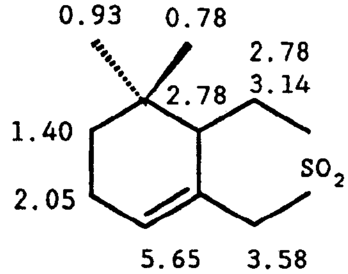

Sulfolène $I_c$

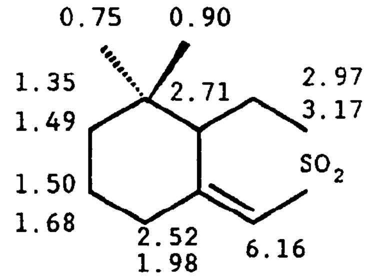

Sulfolène $I_b$

$^{13}$CRMN 90 MHZ

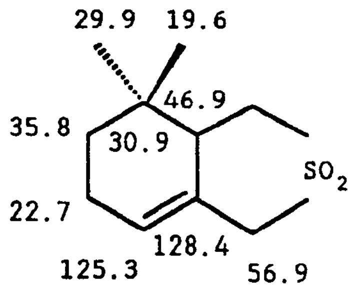

Sulfolène $I_c$

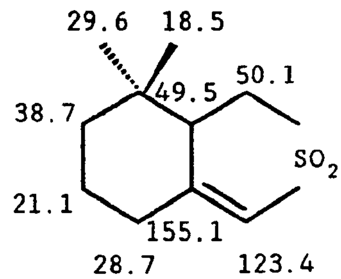

Sulfolène $I_b$

L'analyse a posteriori du mélange brut a permis de déterminer sa composition en masse :

2,5 % hydrocarbures
12,6 % sulfolène du myrcène
84 % sulfones cycliques
$I_a$ 85 %
$I_b$ 10 %
$I_c$ 5 %

soit un taux de transformation =

$$\frac{\text{nombre de moles de produit transformé}}{\text{nombre de moles de matière première introduite}} = 85\%$$

Le rendement par rapport à la sulfone engagée $I_a$ est d'environ 72 %, $I_b$ environ 8,5 %, $I_c$ environ 4,3 %.

<u>EXEMPLE 3</u> <u>PYROLYSE DE Ia</u>

Dans un ballon de 500 ml monté sur évaporateur rotatif, on charge :

- 40 g de sulfolène cyclique $I_a$ (0,2 mole)
- 6 g d'alumine neutre activité I.

Sous vide de 150 mmHg, on porte le ballon en rotation à 200-250°C.

Le distillat est condensé à 20°C et recueilli dans un ballon à 0°C.

On obtient 19,5 g d'un liquide mobile incolore constitué de :

- 17 g δ pyronène
- 2,5 g sulfolène $I_a$

(par analyse $^{1}$H RMN)

Une flash distillation Eb = 102°C/150 mmHg du produit brut permet d'obtenir 15 g de δ pyronène pur analysé par chromatographie en phase vapeur, Rdt : 55 %.

EXEMPLE 4 PYROLYSE DE Ia

Dans un ballon de 100 ml monté sur un évaporateur rotatif, on charge :
– 10 g sulfolène cyclique $I_a$ (0,05 mole)
– 10 g oxyde de calcium en poudre
Sous vide de 40-50 mmHg, on porte le ballon en rotation à 200-220°C, on piège à -80°C 4,2 g de δ pyronène pur par analyse par chromatographie phase gazeuse et RMN (Rdt = 62 %).

EXEMPLE 5

Dans un ballon de 50 ml surmonté d'un condensateur vertical (avec sirculation d'un mélagne réfrigérant maintenu à 0°C) on charge :
– 5 g de sulfolène à 80 % contenant 15 mmoles de forme Ia et 5 mmoles de forme Ic
– 48 mg de ionol
– 104 mg de méthylate de sodium
– 12 ml de gilotherm$^R$ (composé de 75 % de diphényléther et de 25 % de diphényl).
On chauffe 2 heures à 60°C sous pression atmosphérique, on constate la disparition de l'isomère Ic.
On met ensuite, par l'intermédiaire de deux pièges refroidis (carboglace + acétone) sous un vide de 200 mm de mercure. On chauffe progressivement et maintient la température entre 160 et 186°C tout en distillant le δ pyronène formé.
Le rendement en δ pyronène par rappport au sulfolène total est de 76,5 %.

EXEMPLE 6

Dans le même appareillage que précédemment, on charge :
– 10 g de sulfolène (35 mmoles de forme Ia et 10 mmoles de forme Ic et 5 mmoles de forme Ib)
– 112 mg de ionol (0,5 mmole)
– 19,8 g (25 ml) de décanol
– 0,3 g de potasse à 85 % (5 mmoles).
On chauffe à 87°C pendant 30 minutes sous pression atmosphérique. Les isomères Ic et Ib disparaissent totalement.
On procède ensuite comme à l'exemple 5.
Après 1 heure de distillation, le pyronène obtenu est rectifié en présence de bicarbonate de sodium.
Le rendement final en δ pyronène par rapport au sulfolène total est de 81 %.

**Revendications**

**1** - 4,4-diméthyl 2,2-dioxo hexahydrobenzo[c] thiophène de formule (I) :

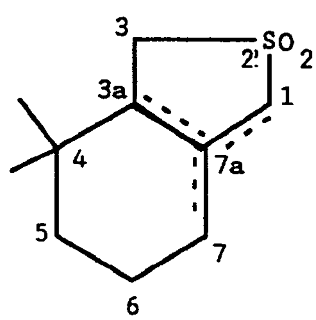

Formule (I)

dans laquelle il existe une double liaison située en position 3a-7a, 1-7a ou 7a-7.

**2** - 4,4-diméthyl 2,2-dioxo 1,3,4,5,6,7-hexahydrobenzo[c] thiophène.

**3** - Procédé de préparation des 4,4-diméthyl 2,2-dioxo hexahydrobenzo[c] thiophènes caractérisé en ce qu'on met en contact le 3-(4-méthyl 3-pentényl)1,1 dioxyde-2,5 dihydro thiophène avec un acide fort.

**4** - Procédé selon la revendication 4 caractérisé en ce que l'acide fort est un acide contenant moins de 5 % d'eau et de préférence moins de 2 %.

**5** - Procédé selon l'une quelconque des revendications 3 ou 4 caractérisé en ce que l'acide fort est choisi

parmi l'acide sulfurique, les acides sulfoniques, l'acide perchlorique et les catalyseurs hétérogènes acides et est de préférence l'acide sulfurique.

**6** - Procédé selon la revendication 5 caractérisé en ce que les acides sulfoniques sont choisis parmi l'acide méthane sulfonique, l'acide paratoluènesulfonique, les résines sulfoniques.

**7** - Procédé selon la revendication 3 caractérisé en ce qu'on ajoute un solvant inerte dans les conditions de la réaction choisis parmi les solvants halogénés, les acides et les esters carboxyliques, les solvants nitrés, le sulfolane.

**8** - Procédé selon la revendication 7 caractérisé en ce que le solvant est le dichlorométhane.

**9** - Procédé selon la revendication 3 caractérisé en ce que le rapport volumique de l'acide sulfurique au thiophène 2,5-dihydro 3-(4-méthyl 3-penténYl)1,1-dioxyde est inférieur à 1 et de préférence compris entre 0,10 et 0,50.

**10** - Utilisation des composés de formule (I) pour la préparation du δ pyronène caractérisé en ce qu'on le chauffe à une température supérieure à 150°C.

**11** - Utilisation des composés de formule (I) selon la revendication 10 caractérisée en ce que l'on chauffe en présence d'un catalyseur basique choisi parmi les hydroxydes alcalins, les carbonates, les oxydes métalliques, les alcoolates.

**12.** Utilisation selon la revendication 11 caractérisée en ce que la quantité de catalyseur varie entre 1 et 100 % en poids par rapport au composé de formule (I).

**13.** Utilisation selon la revendication 11 caractérisée en ce qu'on chauffe à une température comprise entre 150 et 250°C.

Office européen des brevets

# RAPPORT DE RECHERCHE EUROPEENNE

Numero de la demande

EP 91 40 0582

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,Y | US-A-3 176 022 (J.H. BLUMENTHAL)<br>* Colonnes 1,2 *<br>--- | 1-13 | C 07 D 333/72<br>C 07 C 1/32<br>C 07 C 13/19 |
| Y | TETRAHEDRON LETTERS, vol. 24, no. 12, 1983, pages 1247-1250, Pergamon Press, Ltd, GB; R. BLOCH et al.: "A general and stereoselective synthesis of (E,E)-conjugated dienes"<br>* L'article en entier *<br>----- | 10-13 | |

| DOMAINES TECHNIQUES RECHERCHES (Int. Cl.5) |
|---|
| C 07 D 333/00<br>C 07 C 1/00<br>C 07 C 13/00 |

Le présent rapport a été établi pour toutes les revendications

| Lieu de la recherche | Date d'achèvement de la recherche | Examinateur |
|---|---|---|
| LA HAYE | 26-04-1991 | CHOULY J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)